(19) Europäisches Patentamt
European Patent Office
Office européen des brevets

(11) Veröffentlichungsnummer: **0 540 976 A1**

# (12) EUROPÄISCHE PATENTANMELDUNG

(21) Anmeldenummer: **92118290.3**

(22) Anmeldetag: **26.10.92**

(51) Int. Cl.[5]: **C08G 18/80**, E04B 1/66, C04B 41/48, C07C 271/20, C07C 271/24, C07C 311/09, C07C 33/42, C07C 31/40

(30) Priorität: **08.11.91 DE 4136768**

(43) Veröffentlichungstag der Anmeldung: **12.05.93 Patentblatt 93/19**

(84) Benannte Vertragsstaaten: **AT BE CH DE ES FR GB IT LI NL**

(71) Anmelder: **BAYER AG**
**W-5090 Leverkusen 1 Bayerwerk(DE)**

(72) Erfinder: **Kirchmeyer, Stephan, Dr.**
**Heymannstrasse 36**
**W-5090 Leverkusen 1(DE)**
Erfinder: **Müller, Hanns Peter, Dr.**
**Hollweg 20**
**W-5068 Odenthal-Höffe(DE)**
Erfinder: **Kober, Hermann**
**Unterscheider Weg 34**
**W-5060 Bergisch Gladbach 2(DE)**
Erfinder: **Böhm, Stefan, Dr.**
**Carl-Leverkus-Strasse 30**
**W-5090 Leverkusen 1(DE)**
Erfinder: **Pedain, Josef, Dr.**
**Haferkamp 6**
**W-5000 Köln 80(DE)**

## (54) Die Verwendung von Polyisocyanatlösungen zur Imprägnierung von mineralischen Substraten.

(57) Die Verwendung von Lösungen von a) Harnstoffgruppenfreien, fluorenthaltenden Polyisocyanaten in b) gegenüber Isocyanatgruppen inerten Lösungsmitteln zur Imprägnierung von mineralischen Substraten, ausgewählte, hierzu geeignete, fluorenthaltende Polyisocyanate mit einem Fluorgehalt in Form von perfluorierten Alkylgruppen von 15 bis 60 Gew.-% und einem Isocyanatgehalt von 0,5 bis 15 Gew.-%, welche durch Umsetzung von (cyclo)aliphatischen Isocyanaten mit fluoraufweisenden ein- und mehrwertigen Alkoholen hergestellt worden sind, sowie ausgewählte, zur Herstellung derartiger Polyisocyanate geeignete, fluorenthaltende Alkohole.

EP 0 540 976 A1

Die Erfindung betrifft die Verwendung von Lösungen von Harnstoffgruppen-freien, Fluor enthaltenden Polyisocyanaten in inerten Lösungsmitteln zur Imprägnierung von mineralischen Substraten, bevorzugte, hierzu geeignete Polyisocyanate und ausgewählte, zur Herstellung solcher Polyisocyanate geeignete Alkohole.

Mineralische Substrate wie Ziegel, gebrannte Tone, aus natürlichen Gesteinen gewonnene Mauersteine, Keramik, Marmor, Beton, Putze usw. unterliegen im Laufe der Zeit einem Erosionsprozess, welcher im Gesteinsmaterial zu Porösität, Bindemittelverlust und Verlust an mechanischem Zusammenhalt führt. Dieser Erosionsprozess kann durch schädliche Einflüsse aus der Umwelt, wie z.B. Autoabgase oder Säurespuren im Regenwasser stark beschleunigt werden. Im Falle von Bauten oder Gebäuden, welchen eine besondere historische Bedeutung zukommt oder denen ein anderes besonderes Interesse gilt, ist es unumgänglich, diese in ihrem ursprünglichen Aussehen zu erhalten, da eine Erneuerung oder ein Ersatz unerwünscht ist.

Es wurde bereits vorgeschlagen (DE-OS 3 629 061), Polysiloxaneinheiten aufweisende NCO-Prepolymere zur Imprägnierung von mineralischen Substraten einzusetzen. Die empfohlenen Imprägniermittel bewirken zwar eine gute Hydrophobierung der behandelten Substrate, befriedigen jedoch noch nicht bezüglich der in der Praxis ebenso wichtigen Oleophobierung, d.h. sie bewirken keinen voll befriedigenden Schutz gegenüber dem Einfluß von aggressiven, organischen Verunreinigungen, wie sie beispielsweise durch nur unzureichende Verbrennung von Heiz- oder Brennstoffen auf Mineralölbasis in die Umwelt gelangen.

Auch Perfluoralkylgruppen aufweisende Polyisocyanate sind bereits bekannt. So beschreibt beispielsweise DE-A 0 435 641 Harnstoffgruppen und Fluor aufweisende Polyisocyanate, die u.a. auch zur Imprägnierung von Keramik oder Beton emfohlen werden. Aufgrund der durch Reaktion eines Teils der Isocyanatgruppen mit Wasser eingeführten Harnstoffgruppen sind die insbesondere zur Textilimprägnierung emfohlenen Verbindungen jedoch mit dem Nachteil behaftet, daß sie ein wesentlich schlechteres Eindringvermögen in das poröse Gestein und damit in Zusammenhang stehend eine geringere Langzeitwirkung aufweisen. Im übrigen bewirken die Harnstoffgruppen eine Reduzierung der wasserabweisenden Eigenschaften.

Die japanische Patentanmeldung 67-21 331 (C.A. 69 (1968), Referat 11 065a) beschreibt wasserabweisende, Fluor enthaltende Polyisocyanate auf Basis von linearen, teilfluorierten Alkoholen und einem aromatischen Polyisocyanat ohne jeglichen Hinweis auf die Verwendbarkeit dieser Polyisocyanate zur Imprägnierung von mineralischen Substraten.

Die der Erfindung zugrunde liegende Aufgabe bestand darin, ein neues Imprägniermittel für mineralische Substrate zur Verfügung zu stellen, welches gut in die zu schützenden Substrate eindringt und eine lang andauernde Hydro- und Oleophobierung bewirkt. Diese Aufgabe konnte mit der nachstehend näher beschriebenen erfindungsgemäßen Verwendung, insbesondere der erfindungsgemäß bevorzugten Fluor enthaltenden Polyisocyanate gelöst werden.

Gegenstand der Erfindung ist die Verwendung von Lösungen von

a) Harnstoffgruppen-freien, Fluor enthaltenden Polyisocyanaten mit einem Fluorgehalt in Form von perfluorierten Alkylgruppen von 15 bis 60 Gew.-% und einem Isocyanatgehalt von 0,5 bis 15 Gew.-% in

b) gegenüber Isocyanatgruppen inerten Lösungsmitteln,

zur Imprägnierung von mineralischen Substraten.

Gegenstand der Erfindung sind auch die bevorzugten, für die erfindungsgemäße Verwendung geeigneten, Harnstoffgruppen-freien Polyisocyanate, die durch Umsetzung von

i) Polyisocyanaten mit (cyclo)aliphatisch gebundenen Isocyanatgruppen des Molekulargewichtsbereichs 168 bis 1 000 mit unterschüssigen Mengen an

ii) Fluor aufweisenden ein- oder zweiwertigen Alkoholen, ausgewählt aus der Gruppe bestehend aus solchen der Formeln (I) bis (VI)

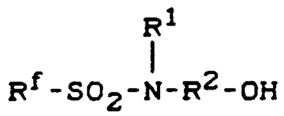 (I)

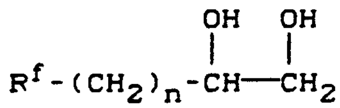 (II)

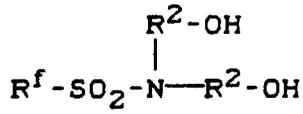 (III)

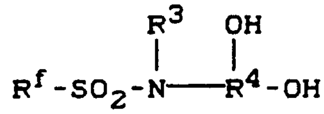 (IV)

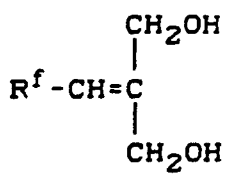 (V)

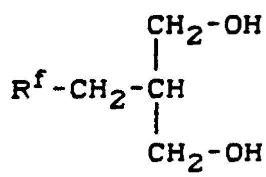 (VI)

gegebenenfalls unter anschließender weiterer Modifizierung der Umsetzungsprodukte und/oder unter anschließender Entfernung von nicht umgesetztem Ausgangsdiisocyanat hergestellt worden sind, wobei in den genannten Formeln

| | |
|---|---|
| $R^f$ | für eine Perfluoralkylgruppe mit 4 bis 16 Kohlenstoffatomen steht, |
| $R^1$ und $R^3$ | für gleiche oder verschiedene Reste stehen und Alkylgruppen mit 1 bis 4 Kohlenstoffatomen bedeuten, |
| $R^2$ | für zweiwertige gesättigte aliphatische Kohlenwasserstoffreste mit 2 bis 4 Kohlenstoffatomen steht, |
| $R^4$ | für einen dreiwertigen aliphatischen Kohlenwasserstoffrest mit 3 bis 4 Kohlenstoffatomen steht und |
| n | für 0, 1 oder 2 steht. |

Gegenstand der Erfindung sind schließlich auch die zur Herstellung solcher Polyisocyanate geeigneten Alkohole der Formeln (V) und (VI).

Zur Herstellung der erfindungsgemäß zu verwendenden, Fluor enthaltenden Polyisocyanate sind beliebige, aus der Polyurethanchemie an sich bekannte Polyisocyanate geeignet, wie sie z.B. von D. Dieterich in "Methoden der Organischen Chemie (Houben-Weyl)", Band E20 (Makromolekulare Stoffe), Herausgeben H. Bartl und J. Falbe, Georg Thieme Verlag, Stuttgart, New York 1987, S. 1587 bis 1595, beschrieben werden, z.B. Ethylendiisocyanat, Tetramethylendiisocyanat, Hexamethylendiisocyanat, 1,12-Dodecandiisocyanat, Cyclobutan-1,3-diisocyanat, Cyclohexan-1,3- und -1,4-Diisocyanat sowie beliebige Gemische dieser Isomeren, 1-Isocyanato-3,3,5-trimethyl-5-isocyanatomethyl-cyclohexan, 2,4- und 2,6-Hexahydrotoluylendiisocyanat sowie beliebige Gemische dieser Isomeren, Hexahydro-1,3- und/oder -1,4-phenylendiisocyanat, Perhydro-2,4'- und/oder -4,4'-diphenylmethandiisocyanat, 1,3- und 1,4-Phenylendiisocyanat, 2,4- und 2,6-Toluylendiisocyanat sowie beliebige Gemische dieser Isomeren,

Diphenylmethan-2,4'- und/oder -4,4'-diisocyanat. Triphenylmethan-4,4',4''-triisocyanat, Polyphenyl-polymethylenpolyisocyanate, wie sie durch die Anilin-Formaldehyd-Kondensation und anschließende Phosgenierung erhalten werden (z.B. GB-PS 874 430, 848 671), Allophanatgruppen aufweisende Polyisocyanate (z.B. GB-PS 994 890, BE-PS 761 626, NL-PS 7 102 524), Isocyanuratgruppen aufweisende Polyisocyanate (z.B. US-PS 3 001 973, DE-PS 1 022 789, 1 222 067, 1 027 394, DE-OS 1 929 034, 2 004 048), Urethangruppen aufweisende Polyisocyanate (z.B. BE-PS 752 261, US-PS 3 394 164, 3 664 457) und Biuretgruppen aufweisende Polyisocyanate (z.B. US-PS 3 124 605, 3 201 372, GB-PS 889 050).

Besonders bevorzugt handelt es sich bei den Ausgangspolyisocyanaten um solche des Molekulargewichtsbereichs 168 bis 1 000, insbesondere 168 bis 300, mit ausschließlich (cyclo)aliphatisch gebundenen Isocyanatgruppen. Zu den bevorzugten Ausgangspolyisocyanaten gehören beispielsweise Hexamethylendiisocyanat (HDI), 4,4'-Diisocyanato-dicyclohexylmethan (HMDI), 1-Isocyanato-3,3,5-trimethyl-5-isocyanatomethyl-cyclohexan (IPDI) sowie Isocyanurat- und gegebenenfalls Urethdiongruppen aufweisende Derivate von HDI und/oder IPDI oder Biuretgruppen aufweisende Polyisocyanate auf Basis von HDI.

Bei den Reaktionspartnern für die Ausgangspolyisocyanate, d.h. den Fluor enthaltenden Alkoholen handelt es sich insbesondere um solche der bereits genannten Formeln (I) bis (VI), wobei es sich bei den Alkoholen (I) bis (IV) um literaturbekannte Verbindungen und bei den Alkoholen (V) bzw. (VI) um neue Substanzen handelt.

In allen diesen Formeln steht $R^f$ für eine perfluorierte Alkylgruppe mit 4 bis 16, vorzugsweise 4 bis 10, Kohlenstoffatomen, während $R^1$, $R^2$, $R^3$, $R^4$ sowie n die bereits genannte Bedeutung haben.

Beispiele für geeignete Alkohole der Formeln (I) bis (IV) sind Perfluor-n-butylethandiol, 3-Perfluor-n-butyl-1,2-propandiol,Perfluor-n-hexylethandiol, 3-Perfluor-n-hexyl-1,2-propandiol, 3-Perfluor-n-heptyl-1,2-propandiol, Perfluoroctylethandiol, 3-Perfluor-n-octyl-1,2-propandiol, Perfluor-n-decylethandiol und 3-Perfluor-n-decyl-1,2-propandiol; Sulfonamidgruppen enthaltende Verbindungen wie z.B. N-Methyl-N-(2-hydroxyethyl)-1-perfluor-n-butansulfonamid, N-Ethyl-N-(2-hydroxyethyl)-1-perfluor-n-butansulfonamid, N-Propyl-N-(2-hydroxyethyl)-1-perfluor-n-butansulfonamid, N-Methyl-N-(2,3-dihydroxypropyl)-1-perfluor-n-butansulfonamid, N,N-Bis(2-hydroxyethyl)-1-perfluor-n-butansulfonamid, N-Methyl-N-(2-hydroxyethyl)-1-perfluor-n-pentansulfonamid, N-Ethyl-N-(2-hydroxyethyl)-1-perfluor-n-pentansulfonamid, N-Methyl-N-(2-hydroxyethyl)-1-perfluor-n-hexansulfonamid, N-Ethyl-N-(2-hydroxyethyl)-1-perfluor-n-hexansulfonamid, N-Propyl-N-(2-hydroxyethyl)-1-perfluor-n-hexansulfonamid, N-Methyl-N-(2-hydroxyethyl)-1-perfluor-n-heptansulfonamid, N-Ethyl-N-(2-hydroxyethyl)-1-perfluor-n-heptansulfonamid, N-Butyl-N-(2-hydroxyethyl)-1-perfluor-n-heptansulfonamid, N-(2-Hydroxyethyl)-1-perfluor-n-octansulfonamid, N-Methyl-N-(2-hydroxyethyl)-1-perfluor-n-octansulfonamid, N-Ethyl-N-(2-hydroxyethyl)-1-perfluor-n-octansulfonamid, N-1-Methylethyl-N-(2-hydroxyethyl)-1-perfluor-n-octansulfonamid, N-Propyl-N-(2-hydroxyethyl)-1-perfluor-n-hexansulfonamid, N-Butyl-N-(2-hydroxyethyl)-1-perfluor-n-octansulfonamid, N,N-Bis(2-hydroxyethyl)-1-perfluor-n-octansulfonamid, N-Methyl-N-(2,3-dihydroxypropyl)-1-perfluor-n-octansulfonamid, N-Ethyl-N-(2,3-dihydroxypropyl)-1-perfluor-n-octansulfonamid, N-Propyl-N-(2,3-dihydroxypropyl)-1-perfluor-n-octansulfonamid, N-Ethyl-N-(2-hydroxyethyl)-1-perfluor-n-nonansulfonamid, N-Propyl-N-(2-hydroxyethyl)-1-perfluor-n-decansulfonamid oder N-Propyl-N-(2-hydroxyethyl)-1-perfluor-n-dodecanonansulfonamid.

Die Herstellung der neuen Alkohole der Formeln (V) und (VI) kann beispielsweise durch Peroxid-initiierte Anlagerung von Perfluoralkyliodiden an 3-Acetoxy-2-acetoxymethyl-1-propen (VII) unter Bildung des Addukts (VIII) und dessen hydrogenolytische und/oder hydrolytische Überführung in die Alkohole (V) bzw. (VI) nach folgendem Formelschema erfolgen:

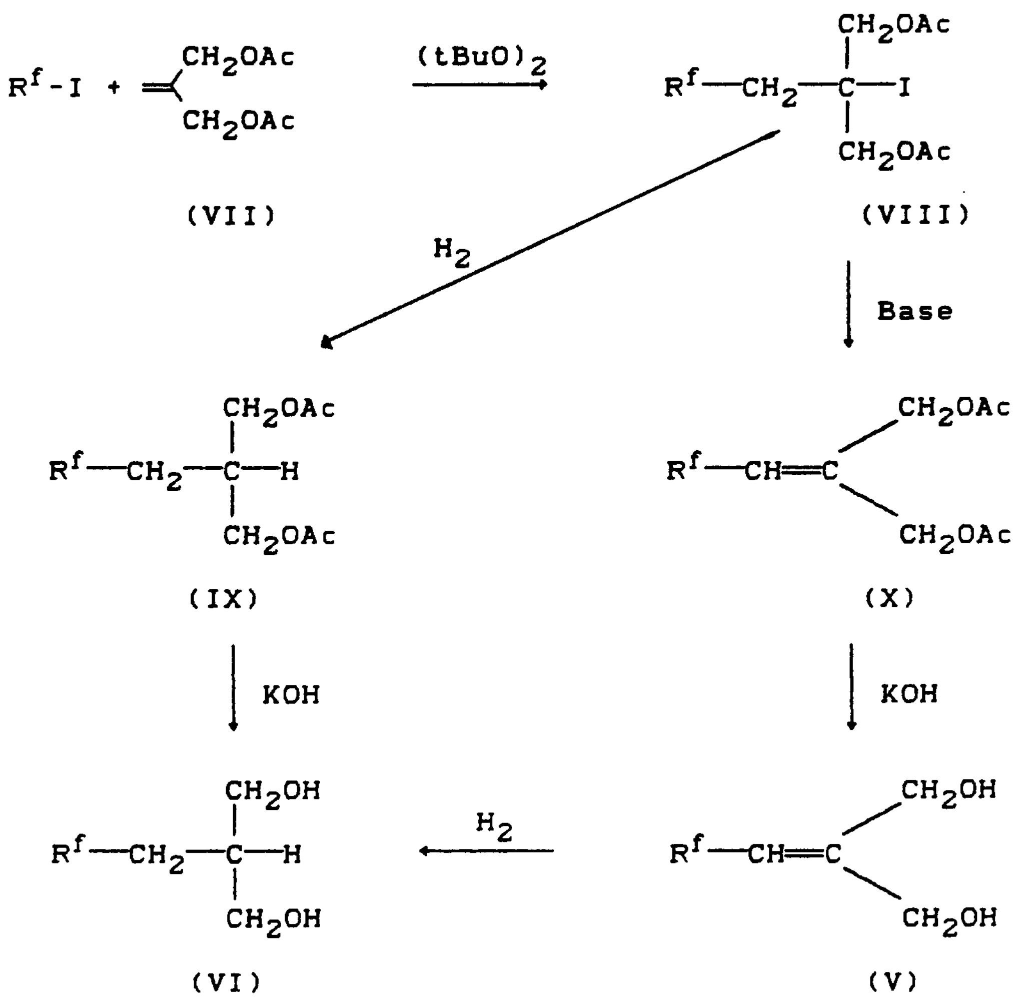


(VII) (VIII) (IX) (X) (VI) (V)

Diese Umsetzungen erfolgen in Analogie zur Synthese von monofunktionellen Alkoholen mit hochfluorierten Substituenten durch radikalische Addition von $R^fI$ an Vinyl- bzw. Allylacetat mit nachfolgender Verseifung und reduktiver Entfernung des Iods, wie sie beispielsweise in US-PS 3 171 861 oder von M.O. Brace in J. Fluorine Chem. 20, 313 (1981) beschrieben ist.

Als Initiator kann beispielsweise Di-tert.-butylperoxid verwendet werden. Die Anlagerungsreaktion unter Bildung des Zwischenprodukts (VIII) erfolgt im allgemeinen innerhalb des Temperaturbereichs von 0 °C bis 150 °C in Substanz. Die Hydrierungsreaktionen (Bildung von IX aus VIII bzw. von VI aus V) können beispielsweise unter Verwendung von Tetrahydrofuran als Reaktionsmedium unter Verwendung üblicher Palladiumkatalysatoren bei 0 °C bis 200 °C, vorzugsweise unter Inertgas durchgeführt werden. Die hydrolytische Spaltung (Bildung von VI aus IX bzw. von V aus X) kann beispielsweise in wäßrigem Metanol unter Verwendung von Alkalihydroxid innerhalb des Temperaturbereichs von 0 °C bis 60 °C in an sich bekannter Weise durchgeführt werden.

Beispiele für auf diese Weise hergestellte, Fluor enthaltende Diole (V) bzw. (VI) sind 1,3-Dihydroxy-2-(1'H,1'H-perfluor-n-pentyl)-propan, 1,3-Dihydroxy-2-(1'H,1'H-perfluor-n-heptyl)-propan, 1,3-Dihydroxy-2-(1'H,1'H-perfluor-n-nonyl)-propan, 1,3-Dihydroxy-2-(1'H,1'H-perfluor-n-undecyl)-propan, 3-Hydroxy-2-hydroxymethyl-1-perfluor-n-butyl-propen, 3-Hydroxy-2-hydroxymethyl-1-perfluor-n-hexyl-propen, 3-Hydroxy-2-hydroxymethyl-1-perfluor-n-octyl-propen, 3-Hydroxy-2-hydroxymethyl-1-perfluor-n-decyl-propen.

Die literaturbekannten, fluorhaltigen Alkohole der Formeln (I) bis (IV), sowie die neuen erfindungsgemäßen, Fluor enthaltenden Alkohole (V) und (VI) stellen die bevorzugten Reaktionspartner zur Herstellung der Fluor enthaltenden Polyisocyanate dar. Außer diesen bevorzugten Ausgangsmaterialien können jedoch auch andere Fluor enthaltende Alkohole wie beispielsweise Perfluoralkylgruppen aufweisende Alkanole mit 4 bis 20 Kohlenstoffatomen und einem Fluorgehalt von 50 bis 75 Gew.-% oder auch Fluor enthaltende

Alkohole wie sie in US-PS 3 478 116, 3 504 016, 3 510 458 oder 3 547 894 beschrieben sind, verwendet werden.

Die Umsetzung zwischen Ausgangspolyisocyanaten und Fluor enthaltenden Alkoholen erfolgt im allgemeinen innerhalb des Temperaturbereichs von 20 bis 140, vorzugsweise 40 bis 120°C unter Einhaltung eines Äquivalentverhältnisses von Isocyanatgruppen zu Hydroxylgruppen von 6:1 bis 1,1:1, vorzugsweise 3:1 bis 1,3:1.

Die Umsetzung wird oftmals in Gegenwart von Katalysatoren für die Isocyanat-Additionsreaktion durchgeführt. Geeignete Katalysatoren sind beispielsweise tert.-Amine wie Triethylamin, N,N-Dimethylbenzylamin, monocyclische oder bicyclische Amidine (DE-OS 1 720 633), Mannichbasen oder organische Zinnverbindungen wie Zinn(II)acetat, Zinn(II)octoat, Dibutylzinnoxid oder Dibutylzinndilaurat.

Bei Verwendung oder Mitverwendung von mehr als difunktionellen Ausgangskomponenten sollte vorzugsweise darauf geachtet werden, daß folgende Gleichung gilt:

$$f = (f_a \, . \, n_a) \leqq 2$$

wobei

- f die mittlere Gesamtfunktionalität an umgesetzten Isocyanat- oder Hydroxylgruppen,
- $f_a$ die mittlere Isocyanat- bzw. Hydroxylfunktionalität der an der Umsetzung beteiligten Ausgangskomponenten und
- $n_a$ den Molenbruch dieser Komponenten, bezogen auf Reaktionsgemisch

bedeuten.

Im allgemeinen werden die Reaktionskomponenten innerhalb des genannten Temperaturbereichs miteinander gerührt, bis der berechnete Gehalt an überschüssigen Isocyanatgruppen erreicht ist.

Zur Vermeidung von unkontrollierbaren Reaktionen kann die Herstellung der fluorierten Isocyanatgruppen aufweisenden Urethane unter vorherigem Zusatz von einem geeigneten, gegenüber Isocyanatgruppen inerten Lösemittel oder Lösemittelgemisch durchgeführt werden. Geeignete Lösungsmittel sind beispielsweise Aceton, 2-Butanon, Cyclohexanon, Isobutylmethylketon, Tetrahydrofuran, Methylacetat, Ethylacetat, Butylacetat, Acetonitril, Chlorbenzol, Chloroform, Dichlormethan, Perchlorethylen, Tetrachlorkohlenstoff, Trichlorethyl, Trichlorethan, Dichlorbenzole, Dimethylformamid, 1-Methyl-2-pyrrolidon, Dimethylacetamid, Dimethylsulfoxid, Dioxan, Hexan, Heptan, Isooctan, Ligroin, Wasch-, Löt-, Leicht-, Lack- oder Testbenzin, Petrolether, Petroleum, Terpentinölersatz, Cyclohexan, Toluol, Xylol, aliphatische oder aromatische Lacklösemittelgemische, Methylcyclohexan, Methylglykolacetat, Methoxypropylacetat oder Mischungen hieraus.

Im Anschluß an die Isocyanat-Additionsreaktion können die resultierenden, Fluor enthaltenden Polyisocyanate einer weiteren Modifizierung und/oder einer Nachbehandlung unterzogen werden.

Unter "weiterer Modifizierung" ist hierbei insbesondere der Einbau von Isocyanurat-, Biuret- oder Allophanatgruppen zu verstehen. Der Einbau von Isocyanuratgruppen kann beispielsweise durch Trimerisierung eines Teils der nach der Isocyanat-Additionsreaktion noch verbleibenden Isocyanatgruppen in Gegenwart von die Trimerisierung von Isocyanatgruppen beschleunigenden Katalysatoren erfolgen. Bei dieser Trimerisierungsreaktion wird im allgemeinen das Gemisch aus Fluor enthaltenden Polyisocyanaten und überschüssigem Ausgangs-Diisocyanat eingesetzt, wie es bei der Isocyanat-Additionsreaktion unmittelbar anfällt. Die Trimerisierung erfolgt vorzugsweise unter Verwendung von üblichen Trimerisierungs-Katalysatoren wie beispielsweise Tetraalkylammoniumhydroxiden der in US-PS 3 487 080 oder DE-OS 2 839 133 beschriebenen Art innerhalb des Temperaturbereichs von 40 bis 120°C bis der gewünschte Trimerisierungsgrad erreicht ist. Der Abbruch der Trimerisierungsreaktion erfolgt in an sich bekannter Weise durch Zugabe eines Katalysatorengifts und/oder durch thermische Zersetzung des Katalysators.

Anstelle des genannten Ausgangsgemisches können bei der Trimerisierungsreaktion auch Gemische aus Fluor enthaltenden Polyisocyanaten der genannten Art mit anderen Diisocyanaten der oben beispielhaft genannten Art und gegebenenfalls außerdem überschüssigem Ausgangspolyisocyanat der bei der Herstellung der Fluor enthaltenden Polyisocyanate eingesetzten Art eingesetzt werden.

Die Biuretisierungsreaktion erfolgt unter Verwendung gleicher Ausgangsgemische, wie sie auch zur Trimerisierung eingesetzt werden, wobei jedoch die Reaktion in Gegenwart von an sich bekannten "Biuretisierungsmitteln" erfolgt. Geeignete Biuretisierungsmittel sind beispielsweise t-Butanol oder Wasser, die dem Gemisch in einer Menge von ca. 4 bis 15 Mol-%, bezogen auf die Isocyanatgruppen des Ausgangsgemisches zugesetzt werden. Die Biuretisierungsreaktion erfolgt durch Erhitzen des Gemisches auf ca. 100 bis 180°C bis zum völligen Verschwinden von die Anwesenheit von Harnstoffgruppen signalisierenden Trübungen.

Die Allophanatisierungsreaktion erfolgt beispielsweise durch Erhitzen eines Ausgangsgemisches der auch bei der Trimerisierung eingesetzten Art auf 60 bis 120 °C, gegebenenfalls in Gegenwart von Katalysatoren wie beispielsweise Benzyltrimethylammoniumhydroxid oder Hydroxyethyltrimethylammoniumhydroxid, die gleichzeitig eine Teiltrimerisierung katalysieren, oder durch eine Säure wie beispielsweise HCl, wobei reine Allophanatprodukten entstehen.

Die Hitzeeinwirkung wird beim gewünschten Allophanatisierungsgrad, der ebenfalls anhand des Isocyanatgehalts ermittelt werden kann, abgebrochen.

Die durch Umsetzung der Ausgangspolyisocyanate mit den Fluor enthaltenden Alkoholen erhaltenen, Fluor enthaltenden Polyisocyanate werden oftmals im Anschluß an ihre Herstellung bzw. im Anschluß an die genannte Modifizierungsreaktion von noch vorliegendem Ausgangspolyisocyanat, beispielsweise durch Dünnschichtverdampfung, befreit.

Die bevorzugten erfindungsgemäß zu verwendenden Polyisocyanate, insbesondere die erfindungsgemäßen Polyisocyanate auf Basis der Fluor enthaltenden Alkohole (I) bis (VI) weisen einen Isocyanatgehalt von 0,5 bis 15, vorzugsweise von 4 bis 12 Gew.-%, ein Molekulargewicht $\overline{M}n$ von 600 bis 4 000, vorzugsweise 1 500 bis 2 500, einen Fluorgehalt in Form von perfluorierten Alkylgruppen von 15 bis 60, vorzugsweise 20 bis 40 und vorzugsweise ein Gehalt an freiem, überschüssigem Ausgangspolyisocyanat von unter 10, vorzugsweise von unter 6 Gew.-% auf.

Die erfindungsgemäß zu verwendenden, Fluor enthaltenden Polyisocyanate können in Form von 1- bis 30-gew.-%igen, vorzugsweise 1- bis 10-gew.-%igen Lösungen in Lösungsmitteln der beispielhaft genannten Art als Imprägniermittel für mineralische Substrate zur Erzeugung von wasser-, öl- oder schmutzabweisenden Schutzschichten verwendet werden. Geeignete mineralische Substrate sind insbesondere Keramik, Beton oder Naturstein. Der Auftrag der Imprägniermittel erfolgt nach an sich bekanntem Verfahren wie Fluten, Spritzen, Rollen, Tauchen oder Streichen.

In den nachfolgenden Beispielen beziehen sich alle Prozentangaben auf das Gewicht.

Beispiele 1 - 3

Herstellung von erfindungsgemäßen Diolen der Formeln (V) und (VI)

Beispiel 1

1,3-Dihydroxy-2-(1'H,1'H-perfluor-n-heptyl)-propan (VI)

1.1 1,3-Diacetoxy-2-iodo-2-(1'H,1'H-perfluor-n-heptyl)-propan (VIII)

1,5 kg (3,36 mol) Perfluor-n-hexyliodid werden mit 25 g (0,17 mol) Di-tert.-butylperoxid und 115 g (0,66 mol) 3-Acetoxy-2-acetoxmethyl-2-propen (VII) versetzt. Dann wird unter Stickstoff auf 115 bis 120 °C erhitzt. Bei dieser Temperatur werden unter Rühren weitere 115 g (0,66 mol) 3-Acetoxy-2-acetoxmethyl-1-propen (VII) zugetropft. Es wird 8 h bei 120 °C nachgerührt, bzw. bis alles 3-Acetoxy-2-acetoxmethyl-1-propen umgesetzt ist. Dann destilliert man das überschüssige Perfluorhexyliodid ab. Man erhält 750 g des Produktes (das sind 91 % d.Th.) als kristallinen Feststoff.

1.2 1,3-Diacetoxy-2-(1'H,1'H-perfluor-n-heptyl)-propan (IX)

507 g (0,82 mol) 1,3-Diacetoxy-2-iodo-2-(1'H,1'H-perfluor-n-heptyl)-propan (VIII) werden in 3 000 ml THF gelöst und mit 91 g (0,9 mol) Triethylamin versetzt. Dann gibt man 20 g Pd/C (10 %ig) zu und hydriert bei 35 °C 5 Stunden. Anschließend wird der Katalysator abfiltriert und das Lösungsmittel abgezogen. Der Rückstand wird in Dichlormethan aufgenommen und mit Wasser, 5 %iger $NaHSO_3$-Lösung und nochmals mit Wasser gewaschen. Nach Trocknen mit $MgSO_4$ wird einrotiert und der Rückstand im Hochvakuum destilliert.
$Kp_{1,3\ mbar}$ = 102 °C.
Ausbeute: 152 g (38 %).

1.3 1,3-Dihydroxy-2-(1'H,1'H-perfluor-n-heptyl)-propan (VI)

150 g (0,3 mol) 1,3-Diacetoxy-2-(1'H,1'H-perfluorheptyl)-propan (IX) werden zu einer Lösung von 84 g (1,45 mol) KOH in 600 ml Methanol gegeben und 16 h bei Raumtemperatur gerührt. Dann gibt man 2 l Wasser zu und extrahiert gründlich mit Methyl-t-butyl-ether (MTBE). Die vereinigten organischen

Phasen werden mit Wasser gewaschen, mit $MgSO_4$ getrocknet und einrotiert. Der Rückstand wird im Hochvakuum destilliert.
$Kp_{0,05\ mbar}$ = 110 bis 111 ° C.
Ausbeute: 89 g (72 %).
Fp.: 74 bis 76 ° C.

Beispiel 2

3 – Hydroxy – 2 – hydroxymethyl – 1 – perfluor – n – hexyl – propen (V)

2.1 3 – Acetoxy – 2 – acetoxymethyl – 1 – perfluor – n – hexyl – propen(X)

2,17 kg (3,5 mol) 1,3 – Diacetoxy – 2 – iodo – 2 – (1'H,1'H – perfluor – n – heptyl) – propan (VIII) werden in 2,5 l Dichlormethan gelöst und bei 0 ° C unter Rühren mit 485 g (3,9 mol) 1,5 – Diazabicyclo[4.3.0]non – 5 – en (DBN) tropfenweise versetzt. Es wird bei 0 ° C nachgerührt, bis laut GC völlständiger Umsatz erreicht ist (ca. 15 h). Dann wird das Reaktionsgemisch mit 10 %iger $NH_4Cl$ – Lösung, 5 %iger $NaHSO_3$ – Lösung und Wasser gewaschen. Nach Trocknen mit $MgSO_4$ wird das Lösungsmittel abgezogen.
Ausbeute: 1,79 kg (94 % d.Th.).

2.2 3 – Hydroxy – 2 – hydroxymethyl – 1 – perfluor – n – hexyl – propen(V)

1,345 kg (2,74 mol) 3 – Acetoxy – 2 – acetoxymethyl – 1 – perfluor – n – hexyl – propen (X) werden in 1,5 l Methanol gelöst und mit einer Lösung von 150 g (2,7 mol) KOH in 400 ml Methanol versetzt. Anschließend läßt man über Nacht bei Raumtemperatur nachrühren. Die Mischung wird mit 2 l ges. $NH_4Cl$ – Lösung versetzt, die Phasen werden getrennt und die wäßrige Phase wird mit MTBE extrahiert. Die vereinigten org. Phasen werden mit $MgSO_4$ getrocknet, eingeengt und im Hochvakuum destilliert.
$Kp_{0,03\ mbar}$ = 105 bis 110 ° C.
Ausbeute: 725 g (66 %).

Beispiel 3

1,3 – Dihydroxy – 2 – (1'H,1'H – perfluor – n – heptyl) – propan (VI)

1,5 kg (3,7 mol) 3 – Hydroxy – 2 – hydroxymethyl – 1 – perfluor – n – hexyl – propen (V) werden in 4 l THF gelöst und in einem Autoklaven mit 375 g Raney – Nickel versetzt. Dann werden 140 bar $H_2$ aufgedrückt und der Ansatz wird insgesamt 70 Stunden bei 120 bis 130 ° C hydriert. Anschließend läßt man abkühlen, entspannt und filtriert den Katalysator ab. Die Lösung wird eingeengt und im Vakuum fraktionierend destilliert.
1. Fraktion:
Nebenprodukt: 1 – Hydroxy – 2 – (1'H,1'H – perfluorheptyl) – propan
$Kp_{12\ mbar}$ = 88 ° C.
Ausbeute: 198 g (14 %).
2. Fraktion:
1,3 – Dihydroxy – 2 – (1'H,1'H – perfluorheptyl) – propan (VI)
$Kp_{0,05}$ mbar = 110 bis 111 ° C.
Fp.: 74 bis 76 ° C.
Ausbeute: 1 105 g (73 %).

Beispiele 4 – 6

Herstellung von erfindungsgemäßen Polyisocyanaten

Beispiel 4

19,76 g (0,04 mol) 1 – Perfluor – n – octyl – 2,3 – dihydroxy – propan und 6,66 g (0,06 mol) Isophorondiisocyanat werden unter Feuchtigkeitsausschluß in einem 100 ml Dreihalskolben mit mechanischer Rührung und Innenthermometer zusammengegeben und bei 120 ° C gerührt, bis das Produkt einen titrierbaren Isocyanatgehalt von 3,0 % aufweist. Man löst das Produkt in 26,42 g Butylacetat und erhält eine klare 50

%ige Lösung eines Fluor enthaltenden Polyisocyanats, welches, bezogen auf Feststoff, einen Fluorgehalt von 57,8 % und einen NCO–Gehalt von 3,0 % aufweist.

Beispiel 5

15,48 g (0,04 mol) Bis–(2–hydroxyethyl)–1–perfluor–n–butansulfonamid und 6,66 g (0,06 mol) Isophorondiisocyanat werden unter Feuchtigkeitsausschluß in einem 100 ml Dreihalskolben mit mechanischer Rührung und Innenthermometer zusammengegeben und bei 120 °C gerührt, bis das Produkt einen titrierbaren Isocyanatgehalt von 4,0 % aufweist. Man löst das Produkt in 22,14 g Butylacetat und erhält eine klare 50 %ige Lösung eines Polyisocyanats, welches, bezogen auf Feststoff, einen Fluorgehalt von 30,9 % und einen NCO–Gehalt von 4,0 % aufweist.

Beispiel 6

49,1 g eines Isocyanuratgruppen aufweisenden Polyisocyanats auf Basis von HDI mit einem NCO–Gehalt von 21,4 % und 44,6 g N–Methyl–N–(2–hydroxyethyl)–1–perfluor–n–butansulfonamid werden unter Feuchtigkeitsausschluß in einem 250 ml Dreihalskolben mit mechanischer Rührung und Innenthermometer zusammengegeben und bei 100 °C gerührt, bis das Produkt einen titrierbaren Isocyanatgehalt von 5,6 % aufweist. Man löst das Produkt in 93,7 g Methoxypropylacetat und erhält eine klare Lösung eines Fluor enthaltenden Polyisocyanats, welches, bezogen auf Feststoff, einen Fluorgehalt von 22,8 % und einen NCO–Gehalt von 5,6 % aufweist.

Beispiel 7

45,6 g eines Biuretgruppen aufweisenden Polyisocyanats auf Basis von HDI mit einem NCO–Gehalt von 23,0 % und 44,6 g N–Methyl–N–(2–hydroxyethyl)–1–perfluor–n–butansulfonamid werden unter Feuchtigkeitsausschluß in einem 250 ml Dreihalskolben mit mechanischer Rührung und Innenthermometer zusammengegeben und bei 100 °C gerührt, bis das Produkt einen titrierbaren Isocyanatgehalt von 5,7 % aufweist. Man löst das Produkt in 93,7 g Methoxypropylacetat und erhält eine klare, leicht gelblich gefärbte 50 %ige Lösung eines Fluor enthaltenden Polyisocyanats, welches, bezogen auf Feststoff, einen Fluorgehalt von 23,7 % und einen NCO–Gehalt von 5,7 % aufweist.

Beispiel 8

49,1 g des Isocyanuratgruppen aufweisenden Polyisocyanats aus Beispiel 6 und 35,7 g N–Methyl–N–(2–hydroxyethyl)–1–perfluor–n–butansulfonamid werden unter Feuchtigkeitsausschluß in einem 250 ml Dreihalskolben mit mechanischer Rührung und Innenthermometer zusammengegeben und bei 100 °C gerührt, bis das Produkt einen titrierbaren Isocyanatgehalt von 7,5 % aufweist. Man löst das Produkt in 198 g Butylacetat und erhält eine klare 30 %ige Lösung eines Fluor enthaltenden Polyisocyanats, welches, bezogen auf Feststoff, einen Fluorgehalt von 20,2 % und einen NCO–Gehalt von 7,5 % aufweist.

Beispiel 9

49,1 g des Isocyanuratgruppen aufweisenden Polyisocyanats aus Beispiel 6 und 53,6 g N–Methyl–N–(2–hydroxyethyl)–1–perfluor–n–butansulfonamid werden unter Feuchtigkeitsausschluß in einem 250 ml Dreihalskolben mit mechanischer Rührung und Innenthermometer zusammengegeben und bei 100 °C gerührt, bis das Produkt einen titrierbaren Isocyanatgehalt von 4,3 % aufweist. Man löst das Produkt in 240 g Butylacetat und erhält eine klare 30 %ige Lösung eines Fluor enthaltenden Polyisocyanats, welches, bezogen auf Feststoff, einen Fluorgehalt von 25,0 % und einen NCO–Gehalt von 4,3 % aufweist.

Beispiel 10

49,1 g des Isocyanuratgruppen aufweisenden Polyisocyanats aus Beispiel 6 und 62,48 g N–Methyl–N–(2–hydroxyethyl)–1–perfluor–n–butansulfonamid werden unter Feuchtigkeitsausschluß in einem 250 ml Dreihalskolben mit mechanischer Rührung und Innenthermometer zusammengegeben und bei 100 °C gerührt, bis das Produkt einen titrierbaren Isocyanatgehalt von 2,8 % aufweist, Man löst das Produkt in 260 g Butylacetat und erhält eine klare 30 %ige Lösung eines Fluor enthaltenden Polyisocyanats, welches, bezogen auf Feststoff, einen Fluorgehalt von 26,0 % und einen NCO–Gehalt von 2,8 % aufweist.

Beispiel 11

40,8 g (0,1 Mol) der Verbindung (VI) aus Beispiel 1 und 33,3 g (0,2 Mol) Isophorondiisocyanat werden unter Feuchtigkeitsausschluß 5 h bei 100 ° C gerührt. Das hochviskose Prepolymer wird 30%ig in Butylacetat gelöst. Die klare Lösung besitzt einen titrierbaren Isocyanatgehalt von 1,66 %.

Beispiel 12 (Verwendung)

Lösungen der Polyisocyanate gemäß Beispiel 4 bis 10 werden durch Zugabe des im jeweiligen Beispiel verwendeten Lösungsmittels auf einen Feststof fgehalt von 10 Gew.-% eingestellt. Anschließend taucht man Keramikplatten einer Größe von 5,0 x 7,0 x 0,5 cm in diese Lösungen für 30 Sekunden und konditioniert 7 Tage bei Raumtemperatur (23 ° C) und 24 Stunden bei 50 ° C. Die Wasseraufnahme wird durch Lagerung unter Wasser über 24 Stunden (Tiefe ca. 1 bis 2 mm) ermittelt. Die Ergebnisse werden in Tabelle 1 berichtet.

Tabelle 1

| Imprägnierung mit Lösung aus | Fluorgehalt im Feststoff | mittlerer Auftrag an Imprägnierung | Wasseraufnahme nach 24 Stunden | Hydrophobierungswirkung **) |
|---|---|---|---|---|
| Beispiel 4 | 57,8 % | 46,0 $g/m^2$ | 0,1 % | 99,5 % |
| Beispiel 5 | 30,9 % | 40,4 $g/m^2$ | 0,55 % | 97,4 % |
| Beispiel 6 | 22,8 % | 30,4 $g/m^2$ | 0,34 % | 98,4 % |
| Beispiel 7 | 23,7 % | 26,9 $g/m^2$ | 0,36 % | 98,3 % |
| Beispiel 8 | 20,2 % | 29,1 $g/m^2$ | 0,40 % | 98,1 % |
| Beispiel 9 | 25,0 % | 28,6 $g/m^2$ | 0,46 % | 97,9 % |
| Beispiel 10 | 26,0 % | 33,9 $g/m^2$ | 0,52 % | 97,6 % |
| Beispiel 11 | 33,3 % | 80,8 $g/m^2$ | 0,43 % | 98,0 % |
| Vergl. *) | keiner | 35,1 $g/m^2$ | 6,13 % | 71,5 % |
| Vergl. (unbehandelte Keramik) | keiner | 0 $g/m^2$ | 21,5 % | 0 % |

*) Imprägnierung mit einer handelsüblichen 5 %igen, wäßrigen Siloxan-Dispersion.

**) Hydrophobierungswirkung

$$= \left(1 - \frac{\text{Wasseraufnahme (Beispiel)}}{\text{Wasseraufnahme (unbehandelt)}}\right) \times 100$$

Beispiel 13 – 16 (Herstellung von erfindungsgemäß verwendbaren Polyisocyanaten)

Beispiel 13

Dieses Beispiel beschreibt die Herstellung eines Urethangruppenhaltigen Diisocyanats aus Hexamethylendiisocyanat (HDI) und der Verbindung V aus Beispiel 2.2.

In 101 g HDI trägt man unter gutem Rühren und Erhitzen auf 100 °C 40,8 g Verbindung V ein und erhitzt 2 Stunden auf 100 °C. Danach destilliert man überschüssiges HDI in einem Dünnschichtverdampfer bei 140 °C ab. Als nichtdestillierbaren Rückstand erhält man 71 g eines im wesentlichen difunktionellen Isocyanats als viskose klare Flüssigkeit.

Beispiel 14

Dieses Beispiel beschreibt die Herstellung eines Urethangruppen und Isocyanatgruppen aufweisenden Polyisocyanat aus Hexamethylendiisocyanat (HDI) und der Verbindung V aus Beispiel 2.2.

840 g HDI werden auf 65 °C erhitzt und tropfenweise mit 7,5 g einer Lösung von Benzyltrimethylammoniumhydroxid in 2-Ethylhexandiol-1,3 (0,5%ig) versetzt. Es beginnt eine leicht exotherme Umsetzung, wobei die Temperatur auf 70 °C ansteigt. Nach ca. 5 Stunden unterbricht man die Trimerisierungsreaktion durch Zugabe von 0,075 g einer Abstopperlösung (Dibutylphosphat 25%ig in HDI).

Anschließend fügt man 204 g der Substanz V aus Beispiel 2.2 zu und erhitzt noch 2 Stunden auf 100 °C. Das klare Umsetzungsgemisch wird einer Dünnschichtdestillation unterworfen zur Abtrennung von freiem HDI. Das erhaltene Produkt ist eine klare Flüssigkeit mit einer Viskosität von 4400 mPas/23 °C, einem NCO-Gehalt von 13,4 % und einem Fluorgehalt von 25,3 %.

Beispiel 15

Aus 840 g HDI und 161 g der Substanz

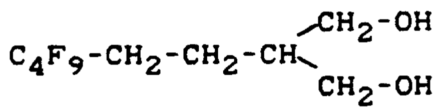

wird wie in Beispiel 14 angegeben ein Isocyanurat- und Urethangruppenhaltiges Polyisocyanat hergestellt. Nach Abdestillieren von überschüssigem HDI erhält man 527 g einer viskosen Flüssigkeit mit folgenden Kennzeichen:
NCO: 14,9 %
Fluorgehalt: 16,2 %
Viskosität: 7000 mPas/23 °C

Beispiel 16

Aus 840 g HDI und 204 g Substanz VI aus Beispiel 1.3 wird wie in Beispiel 14 angegeben ein Isocyanurat- und Urethangruppen-haltiges Polyisocyanat hergestellt. Nach Entfernung von Überschüssigem HDI erhält man 540 g einer viskosen Flüssigkeit mit folgenden Kennzeichen:
NCO: 14,0 %
Fluorgehalt: 22,9 %
Viskosität: 7450 mPas/23 °C

**Patentansprüche**

**1.** Verwendung von Lösungen von
a) Harnstoffgruppen-freien, Fluor enthaltenden Polyisocyanaten mit einem Fluorgehalt in Form von perfluorierten Alkylgruppen von 15 bis 60 Gew.-% und einem Isocyanatgehalt von 0,5 bis 15 Gew.-% in
b) gegenüber Isocyanatgruppen inerten Lösungsmitteln,
zur Imprägnierung von mineralischen Substraten.

**2.** Verwendung gemäß Anspruch 1, dadurch gekennzeichnet, daß es sich bei den Fluor enthaltenden Polyisocyanaten um Urethangruppen aufweisende Polyisocyanate handelt, die durch Umsetzung von
i) Polyisocyanaten mit (cyclo)aliphatisch gebundenen Isocyanatgruppen des Molekulargewichtsbereichs 168 bis 1 000 mit unterschüssigen Mengen an
ii) Fluor aufweisenden ein- oder zweiwertigen Alkoholen, ausgewählt aus der Gruppe bestehend aus solchen der Formeln (I) bis (VI)

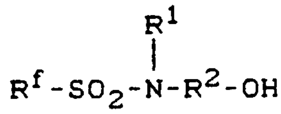 (I)

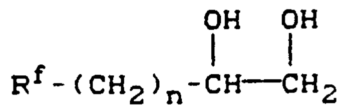 (II)

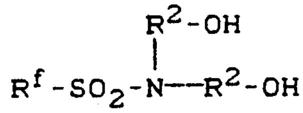 (III)

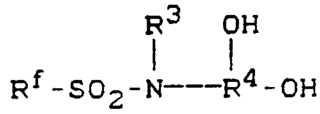 (IV)

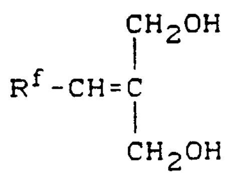 (V)

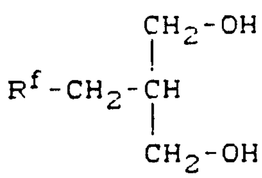 (VI)

gegebenenfalls unter anschließender weiterer Modifizierung der Umsetzungsprodukte und/oder unter anschließender Entfernung von nicht umgesetztem Ausgangsdiisocyanat hergestellt worden sind, wobei in den genannten Formeln

| | |
|---|---|
| $R^f$ | für eine Perfluoralkylgruppe mit 4 bis 16 Kohlenstoffatomen steht, |
| $R^1$ und $R^3$ | für gleiche oder verschiedene Reste stehen und Alkylgruppen mit 1 bis 4 Kohlenstoffatomen bedeuten, |
| $R^2$ | für zweiwertige gesättigte aliphatische Kohlenwasserstoffreste mit 2 bis 4 Kohlenstoffatomen steht, |
| $R^4$ | für einen dreiwertigen aliphatischen Kohlenwasserstoffrest mit 3 bis 4 Kohlenstoffatomen steht und |
| n | für 0, 1 oder 2 steht. |

**3.** Fluor enthaltende Polyisocyanate mit einem Fluorgehalt in Form von perfluorierten Alkylgruppen von 15 bis 60 Gew.-% und einem Isocyanatgehalt von 0,5 bis 15 Gew.-%, dadurch gekennzeichnet, daß sie Urethangruppen aufweisende Umsetzungsprodukte darstellen, die durch Umsetzung von

i) Polyisocyanaten mit (cyclo)aliphatisch gebundenen Isocyanatgruppen des Molekulargewichtsbereichs 168 bis 1 000 mit unterschüssigen Mengen an

ii) Fluor aufweisenden ein- oder zweiwertigen Alkoholen, ausgewählt aus der Gruppe bestehend aus solchen der Formeln (I) bis (VI)

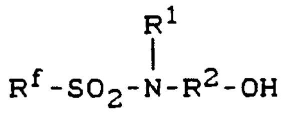

(I)

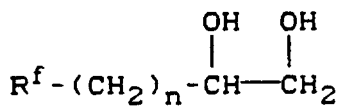

(II)

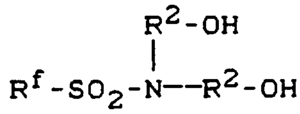

(III)

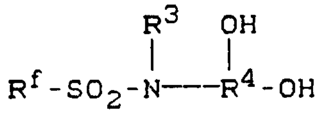

(IV)

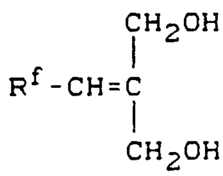

(V)

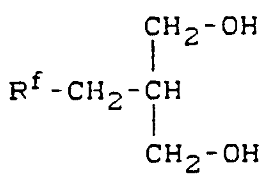

(VI)

gegebenenfalls unter anschließender weiterer Modifizierung der Umsetzungsprodukte und/oder unter anschließender Entfernung von nicht umgesetztem Ausgangsdiisocyanat hergestellt worden sind, wobei $R^f$, $R^1$, $R^2$, $R^3$, $R^4$ und n die in Anspruch 2 genannte Bedeutung haben.

4. Zur Herstellung von Polyisocyanaten gemäß Anspruch 3 geeignete Alkohole der Formeln

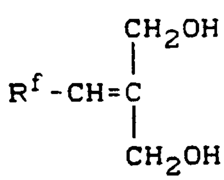

(V)

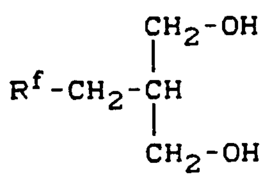

(VI)

für welche $R^f$ die in Anspruch 2 genannte Bedeutung haben.

Europäisches Patentamt

# EUROPÄISCHER RECHERCHENBERICHT

Nummer der Anmeldung

EP 92 11 8290

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| X | DE-A-1 418 985 (MINNESOTA MINING)<br>* Anspruch 1 *<br>* Seite 8, Absatz 2 - Seite 10, Absatz 1 *<br>* Seite 13, Zeile 1 - Zeile 20 * | 1-3 | C08G18/80<br>E04B1/66<br>C04B41/48<br>C07C271/20<br>C07C271/24<br>C07C311/09<br>C07C33/42<br>C07C31/40 |
| X | US-A-4 046 944 (K.F. MUELLER ET AL.)<br>* Ansprüche 1,2 *<br>* Spalte 3, Zeile 24 - Spalte 4, Zeile 66 *<br>* Spalte 5, Zeile 46 - Spalte 6, Zeile 22 *<br>* Spalte 7, Zeile 38 - Zeile 46 * | 3 | |
| X | EP-A-0 127 061 (BAYER)<br>* Ansprüche 1,3,5,6 *<br>* Seite 6, Zeile 25 - Seite 7, Zeile 25 * | 3 | |
| A | EP-A-0 283 892 (ASAHI GLASS CO.)<br>* Ansprüche 1,3,4,11 *<br>* Spalte 6, Zeile 27 - Zeile 53 *<br>* Spalte 8, Zeile 39 - Zeile 57 * | 1 | **RECHERCHIERTE SACHGEBIETE (Int. Cl.5 )** |
| A | EP-A-0 405 534 (SYREMONT)<br>* Ansprüche 1,4 *<br>* Seite 2, Zeile 1 - Zeile 2 *<br>* Seite 4, Zeile 41 - Zeile 47 * | 1 | C08G<br>E04B<br>C04B<br>C07C |
| A | US-A-3 478 116 (K.C. SMELTZ)<br>* Ansprüche 1-3 *<br>* Spalte 6, Zeile 26 - Spalte 7, Zeile 34 *<br>* Spalte 16, Zeile 22 - Zeile 36 *<br>* Beispiel 32 * | 4 | |

Der vorliegende Recherchenbericht wurde für alle Patentansprüche erstellt

| Recherchenort | Abschlußdatum der Recherche | Prüfer |
|---|---|---|
| DEN HAAG | 15 FEBRUAR 1993 | VAN PUYMBROECK M. A. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)